# EUROPÄISCHE PATENTANMELDUNG

(11) **EP 0 818 203 A1**
(43) Veröffentlichungstag der Anmeldung: **14.01.1998**
(21) Anmeldenummer: 97107315.0
(22) Anmeldetag: 02.05.1997
(51) Int. Cl.: A61K 35/78

(54) **Verwendung von Bärlauch zur Herstellung eines Arzneimittels zur Behandlung und/oder Prophylaxe von Hyperkeratosen**

(30) Priorität: 12.07.1996 DE 19628284
(71) Anmelder: PANDALIS, Georgios, D-49219 Glandorf (DE)
(72) Erfinder: Pandalis, Georgios, Dr., D-49219 Glandorf (DE); Heymann, Eberhard, Prof. Dr., D-49074 Osnabrück (DE)
(74) Vertreter: Grünecker, Kinkeldey, Stockmair & Schwanhäusser Anwaltssozietät

(57) **Zusammenfassung**

Die Erfindung betrifft die Verwendung von Allium ursinum L. (Bärlauch) zur Behandlung und/oder Prophylaxe von Hyperkeratosen.

## Beschreibung

Die Erfindung betrifft die Verwendung von Allium ursinum L. (Bärlauch).

Allium ursinum L. (Bärlauch, Bärenlauch, wilder Knoblauch) gehört, wie der Knoblauch, zur Familie der Liliaceae. Die Pflanze ist u.a. in fast ganz Europa beheimatet und wird auch als Heil-, Gewürz- und Gemüsepflanze kultiviert. Für Arzneimittel werden die Bärlauchzwiebel (Allii ursini bulbus), das Bärlauchkraut (Allii ursini herba) sowie die ganze, frische zur Blütezeit gesammelte Pflanze für homöopathische Zwecke verwendet. Bärlauchblätter werden darüber hinaus auch für allophatische Zwecke genutzt. Sie werden, wie das Kraut, nicht nur während, sondern auch vor oder nach der Blütezeit gepflückt. Die Inhaltsstoffe der Zwiebel und des Krautes sind als identisch anzusehen. (S. Hagers Handbuch der Pharmazeutischen Praxis, 4. Band, 5. Auflage, Springer Verlag, im Druck).

In der Bärlauchzwiebel lassen sich abhängig von Extraktionsmedium und -verfahren unterschiedliche Muster an genuinen und nicht genuin enthaltenden Inhaltsstoffen nachweisen. Dazu gehören Cysteinsulfoxide, wie S-Methyl-L-(+)-cysteinsulfoxid und S-Allyl-L-(+)-cysteinsulfoxid (Alliin) und Thiosulfinate, die duch Fermentation aus den Cysteinsulfoxiden gebildet werden. Beispiele für die Thiosulfinate sind Allylmethylthiosulfinat bzw. Methylallylthiosulfinat, Diallylthiosulfinat (Allicin) und Dimethylthiosulfinat. Weitere, nicht genuin in der Bärlauchzwiebel enthaltene Stoffe sind Dithiine, wie Vinyldithiine, Ajoen (4,5,9-Trithiododeca-1,6,11-trien-9-oxid), das durch Selbstkondensation von Allicin entsteht, sowie Ajoenhomologe. Ferner werden als wasserdampfflüchtige Bestandteile (12% bezogen auf die frische Bärlauchzwiebel) Methylallyltrisulfid, das den Hauptbestandteil des Wasserdampfdestillats bildet, sowie geringe Mengen an Diallyldisulfid und Diallyltrisulfid erhalten.

Diese Stoffe sind ebenfalls nicht genuin in der Bärlauchzwiebel enthalten. Weitere, genuin in der Bärlauchzwiebel enthaltende Stoffe sind beispielsweise die freien Aminosäuren L-Arginin, L-Asparaginsäure, L-Glutaminsäure, L-Asparagin, L-Glutamin, L-Glycin, L-Threonin und L-Alanin.

Die frische Zwiebel enthält nach der Fermentation etwa 0,05 bis 0,12% Allicin.

Untersuchte Wirkungen der Bärlauchzwiebel sind eine Lipoxygenase- und Cyclooxygenasehemmung, sowie eine Hemmung im PAF-Thrombozytenaggregationstest.

Frische Bärlauchblätter enthalten nach der Fermentation etwa 0,005% Allicin, getrocknete etwa 0,07%. Ferner enthalten frische Bärlauchblätter etwa 0,007% wasserdampfflüchtige Bestandteile, die den wasserdampfflüchtigen Bestandteilen der Bärlauchzwiebel entsprechen.

Volkstümlich werden Bärlauchblätter und -zwiebeln bei Magen-Darm-Störungen, Gärungsdyspepsien, Flatuleszenz, gegen Bluthochdruck und Arteriosklerose eingesetzt. Äußerlich finden sie Anwendung bei Hautausschlägen.

Die Urtinktur aus ganzen, frischen, zu Beginn der Blütezeit gesammelten Bärlauchpflanzen HAB1 ist eine goldgelbe Flüssigkeit mit Geruch und Geschmack nach Knoblauch HAB1. Die Anwendungsgebiete entsprechen dem homöopathischen Arzneimittelbild. Dazu gehört beispielsweise Verdauungsschwäche.

Es ist bereits bekannt, Bärlauch zur Behandlung oder Prophylaxe von Herzrhythmusstörungen (DE-B-42 30 189) sowie zur Behandlung oder Prophylaxe von Durchblutungsstörungen (DE-B-42 30 188) einzusetzen. Die Epidermis der menschlichen Haut kann einer übermäßig starken Hornbildung (Hyperkeratose) ausgesetzt sein, die zu Entzündungen bzw. Wucherungen führen kann (Proliferationshyperkeratose). Bekannte Beispiele solcher Verhornungsstörungen sind Hautschwielen oder Hühneraugen. Für die Behandlung deartiger Verhornungsstörungen sind verschiedene Verfahren vorgeschlagen worden, z.B. die Entfernung überschüssiger Hornhaut mit hauterweichenden Mitteln, wie Salicylsäure in Verbindung mit mechanischen Verfahren. Die Regulation des Hornhautwachstums wird in der dermatologischen Praxis zur Zeit mit Retinsäure oder Derivaten davon angestrebt. Die bisherigen Verfahren sind aber nicht frei von störenden Nebenwirkungen und sind nicht in jedem Fall wirksam.

Aufgabe der vorliegenden Erfindung ist es, ein Mittel auf natürlicher Basis, das toxikologisch unbedenklich ist, zur Prophylaxe oder Behandlung von Verhornungsstörungen der Haut zur Verfügung zu stellen.

Diese Aufgabe wird erfindungsgemäß gelöst durch die Verwendung von Allium ursinum L. zur Prophylaxe und/oder Behandlung (Bekämpfung) von Hyperkeratosen, insbesondere von Proliferationshyperkeratosen, wie follikuläre Keratosen (Acne vulgaris, Callositas (Hautschwielen), Klavus (Hühnerauge) und Psoriasis vulgaris (Schuppenflechte).

Dafür kann die ganze Pflanze in frischem oder getrocknetem Zustand, sowie einzelne frische oder getrocknete Pflanzenteile, wie die Bärlauchzwiebel oder das Bärlauchkraut oder nur die Blätter, verabreicht werden. Weiterhin sind auch verschiedene Extrakte der Bärlauchzwiebel und/oder des -krauts, die durch Wasserdampfdestillation erhaltenen Fraktionen und die Urtinktur sowie Mischungen daraus wirksam.

Erfindungsgemäß wird Bärlauch bevorzugt in Form üblicher pharmazeutischer Zubereitungen, wie Lösungen, Konzentraten, Tabletten oder Kapseln, oral verabreicht. Besonders bevorzugt ist die Verabreichung von getrockneten, pulverisierten Bärlauchblättern. Das Pulver wird mit Wasser eingenommen. Die benötigte Dosierung der Wirkstoffe wird in Abhängigkeit von üblichen Faktoren, wie der Natur und Schwere der Erkrankung und dem Körpergewicht des Patienten, vom Arzt festgelegt. Üblicherweise wird der Bärlauch in einer Dosierung von 1 bis 5 g/Tag verabreicht.

Zusätzlich oder alternativ zur oralen Verabreichung wird der Bärlauch kutan, d.h. äußerlich durch Auftragen auf die Haut, insbesondere in Form einer dermatologischen Zubereitung, verabreicht. Eine solche dermatologische Zubereitung enthält neben üblichen Inhaltsstoffen erfindungsgemäß einen Bärlauch-Extrakt, vorzugsweise einen wässrigen Estrakt aus Bärlauchblättern. Der Anteil der Bärlauch-Inhaltsstoffe beträgt vorzugsweise 0,01 bis 1 Gewichtsprozent, bezogen auf das Gesamtgewicht der Zubereitung. Je nach Art der Verhornungsstörungen kann die Konzentration an Bärlauch-Extrakt variiert werden, wobei die höchste Konzentration bei Callositas und Klavus anzuwenden ist.

Dermatologische Zubereitungen umfassen beispielsweise Umschläge, Salben und Pasten, vorzugsweise aber Cremes. Bei topischer Applikation solcher Zubereitungen, enthaltend einen wässrigen Extrakt aus Bärlauchblättern, werden die Wirkstoffe in einer Konzentration durch die Hornhaut resorbiert, die ausreicht, eine übermäßige Verhornung auf ein Normalmaß zu senken.

Als besonders vorteilhaft erweist sich die Anwendung einer Creme, die folgendermaßen zusammengesetzt ist:

Die Wasserphase enthält 5 - 100% Bärlauchextrakt. Sie kann bis zu 10% Glycerin oder ein anderes wasserbindendes Mittel enthalten, so daß auf der Haut zusätzlich die Vorteile einer wasserbindenden Hautcreme erreicht werden. Die Wasserphase wird z.B. mit NaH₂PO₄ auf einen hauffreundlichen pH-Wert von 5,5 bis 6,0 eingestellt.

Die Lipidphase besteht aus 8-20% eines gegen Oxidation weitgehend unempfindlichen Öls (z.B. Olivenöl, Jojobaöl oder Paraffinöl). Alternativ oder zusätzlich zu diesem Öl kann auch Isopropylmyristat verwendet werden, das bei einer Resorption durch die Hornhaut in die ungiftigen Komponenten Myristinsäure und Isopropanol hydrolysiert wird. Ferner kann die Lipidphase natürliche oder synthetische Emulgatoren enthalten, die für pflegende Hautcremes gebräuchlich sind. Ein Zusatz von bis zu 5% Tocopherol (Vitamin E) oder Tocopherolacetat stabilisiert gegen Oxidation und begünstigt die hautpflegende Wirkung.

Wasserphase (etwa 2/3) und Ölphase (etwa 1/3) werden bei Temperaturen von maximal 60° zu einer Emulsion verrührt. Zur Verhinderung des vorzeitigen Verderbs können Lebensmittelkonservierungsstoffe, wie PHB(Polyhydroxybuttersäure-)Ester, in den für Hautcremes üblichen Mengen zugesetzt werden.

Um Allergien zu vermeiden, sollten die Präparate gegebenenfalls nur ganz leicht parfümiert werden, z.B. durch Zusatz von 0,1% Phenylethanol zur Ölphase.

Zur Herstellung von Bärlauch-Extrakt enthaltenden Hautcremes wird der Bärlauch-Extrakt zunächst wie folgt gewonnen: 100 g Bärlauch-Granulat Dr. Pandalis werden fein pulverisiert und dann 2 h mit 2 l einer wässrigen Lösung bei 60°C geschüttelt. Die wässrige Lösung enthält pro l 250 mg CaCl₂, 400 mg KCl, 200 mg MgSO₄·7H₂O, 6800 mg NaCl, 160 mg NaH₂PO₄·2H₂O, 1000 mg Glucose und 650 mg einer Mischung von Aminosäuren. Der pH der Extraktionslösung liegt bei 5,0 bis 6,0. Der primäre Extrakt wird durch Zentrifugation geklärt und anschließend steril filtriert.

Die folgenden Versuche zur pflegenden und entschuppenden Behandlung von Psoriasis-Plaques mit "Bärlauch-Gel" wurden in einer Fachklinik für Hauterkrankungen durchgeführt:

Der Versuch wurde mit vier Probanden durchgeführt, die unter chronisch stationären Schuppenflechte-Herden litten.

### Prüfungsverlauf:

Bei den vier Probanden bestanden unbehandelte, durch starke Schuppenauflagerung gekennzeichnete Psoriasis-Herde. Die Probanden wurden angehalten, über sechs Wochen Bärlauch-Gel (Bärlauch-Extrakt in Gel-Grundlage) 2 x täglich auf die zu behandelnden Herde aufzutragen. Daneben wurden keine Bestrahlungstherapien oder andere systemische Behandlungen durchgeführt. Es erfolgte eine Photodokumentation vor Beginn der Anwendung, bei 2 Probanden nach drei Wochen sowie nach sechs Wochen.

### Ergebnisse:

Die klinische Untersuchung sowie die Auswertung der Photodokumentation ergab eine deutliche Verminderung der Schuppenauflagerung in zwei Fällen. In einem Fall wurde eine leichte Verminderung der Schuppen festgestellt, jedoch bei Zunahme der entzündlichen Reaktionen. In einem weiteren Fall ergab sich ein unveränderter Befund.

### Beurteilung:

Aus dermatologischer Sicht ist eine Tendenz zur Verringerung der übermäßigen Hornbildung erkennbar, die über das Maß einer rein mechanischen oder durch die Gel-Grundlage bedingten Ablösung der Schuppung hinausgeht.

## Patentansprüche

1. Verwendung von Allium ursinum L. (Bärlauch) zur Herstellung eines Arzneimittels zur Behandlung und/oder Prophylaxe von Hyperkeratosen.

2. Verwendung nach Anspruch 1 zur Behandlung und/oder Prophylaxe von follikulären Keratosen, Callositas, Klavus oder Psoriasis vulgaris.

3. Verwendung nach Anspruch 1 oder 2, **dadurch gekennzeichnet**, daß der Bärlauch in oraler und/oder kutaner Form verabreicht wird.

4. Verwendung nach Anspruch 3, **dadurch gekennzeichnet**, daß der Bärlauch oral in Form getrockneter, pulverisierter Blätter und/oder kutan in Form einer dermatologischen Zubereitung verabreicht wird.

5. Verwendung nach Anspruch 4, **dadurch gekennzeichnet**, daß die dermatologische Zubereitung einen Bärlauch-Extrakt enthält.

6. Verwendung nach Anspruch 5, **dadurch gekennzeichnet**, daß der Extrakt ein wässriger Extrakt aus Bärlauchblättern ist.

7. Verwendung nach einem der Ansprüche 4 bis 6, **dadurch gekennzeichnet**, daß die Zubereitung eine Creme ist.
